# FASCICULE DE BREVET EUROPEEN

(11) **EP 2 762 879 B1**
(45) Date de publication et mention de la délivrance du brevet: **07.06.2017**
(21) Numéro de dépôt: 14151491.9
(22) Date de dépôt: 16.01.2014
(51) Int. Cl.: G01N 33/42, G01N 33/38, G01S 13/89, B32B 11/04, B32B 3/08

(54) **MEMBRANE OU GÉOMEMBRANE BITUMINEUSE PRÉFABRIQUÉE POUR OUVRAGE DE GÉNIE CIVIL**
BITUMINÖSE (GEOLOGISCHE) FERTIG-DICHTUNGSBAHN FÜR TIEFBAUTEN
PREFABRICATED BITUMINOUS MEMBRANE OR GEOMEMBRANE FOR CIVIL ENGINEERING STRUCTURE

(30) Priorité: 30.01.2013 FR 1350794
(43) Date de publication de la demande: 06.08.2014
(73) Titulaire: ICOPAL SAS, 92184 Antony Cedex (FR)
(72) Inventeur: Benchet, Renaud, 92120 MONTROUGE (FR)
(74) Mandataire: Lenne, Laurence

(56) Documents cités:
- WO-A1-2004/083555
- CN-U- 201 686 920

## Description

L'invention concerne une membrane ou une géomembrane bitumineuse préfabriquée en particulier pour bâtiments, ouvrages d'art, ouvrages enterrés, ouvrages hydrauliques ou ouvrages de protection de l'environnement.

Par membrane bitumineuse, il est entendu, dans ce qui suit, une membrane à base de bitume ou une géomembrane à base de bitume. De telles membranes sont en général sous forme de bandes ou lés souples d'une longueur de 5 à 100 m et de largeur comprise entre 0,2 et 4 m, conditionnés en rouleau.

En général, une telle membrane comporte une armature en non-tissé de polyester imprégnée à coeur de bitume modifié par des élastomères, une couche supérieure de bitume d'enduction de surface et une couche inférieure de bitume d'enduction de sous-face.

Dans le cadre des ouvrages d'art de type pont ou équivalents, un contrôle par radar est fréquemment utilisé pour déterminer l'épaisseur d'asphalte et/ou d'enrobé à raboter au-dessus de la couche d'étanchéité lors d'une remise en état de la chaussée, par détermination de la position de cette couche d'étanchéité sous la couche d'asphalte et/ou d'enrobé, afin de ne pas l'endommager lors de ce rabotage précédent la remise en état.

Grâce à ce moyen de détection non destructif, utilisant la réflexion d'ondes électromagnétiques sur les interfaces des couches présentant des caractéristiques électromagnétiques différentes, une image continue de la structure auscultée peut être déduite. Cependant, les ondes électromagnétiques se propagent dans la structure et ne sont que partiellement réfléchies aux interfaces des couches.

Par contre, sur des matériaux métalliques, la réflexion des ondes électromagnétiques est totale et le signal obtenu est donc très caractéristique.

Il peut donc être envisagé de disposer sur une face de la couche d'étanchéité un élément métallique où les ondes électromagnétiques sont totalement réfléchies lors d'un contrôle radar et en conséquence le signal de détermination de la position de la couche d'étanchéité particulièrement significatif.

Le document de brevet CN 201 686 920 décrit un procédé de contrôle par radar d'un ouvrage de génie civil consistant à disposer sur la face inférieure des différentes couches d'asphalte associées à leur couche de liaison ou d'étanchéité, plusieurs fils de métal d'un diamètre d'environ 1mm.

Cependant, un tel procédé pose les problèmes techniques suivants.

Le contrôle par radar est classiquement également utilisé pour le contrôle de la structure porteuse en béton, par exemple du tablier d'un pont, en particulier pour contrôler le positionnement des armatures, et cet autre contrôle s'avère difficile voire même impossible, si un maillage de fils métalliques bloque les ondes électromagnétiques au-dessus de cette structure porteuse.

Il serait alors envisageable de disposer de tels fils métalliques avec un écart suffisant sur la face supérieure de la membrane ou géomembrane préfabriquée, afin de répondre au problème évoqué plus haut de détermination de la position de cette couche d'étanchéité sous la couche d'asphalte et/ou d'enrobé, afin de ne pas l'endommager lors du rabotage de la couche d'asphalte et/ou d'enrobé.

Mais cette solution pose un autre problème technique. Lors du compactage de la couche d'asphalte et/ou d'enrobé, au moment de sa mise en place, le fil métallique peut être déchiré, déformé ou déplacé ce qui le rend en général difficilement détectable par radar.

L'invention résout ce problème en proposant une membrane ou géomembrane préfabriquée assurant un contrôle par radar fiable, tout en assurant une stabilité de la structure totale.

Pour ce faire, l'invention concerne une membrane ou géomembrane bitumineuse préfabriquée pour la mise en oeuvre d'un procédé de contrôle par radar, comportant au moins un élément métallique, caractérisée en ce que ledit élément métallique est constitué d'un galon unique d'une largeur comprise entre 15 et 20 mm noyé dans la membrane à une profondeur sensiblement constante.

L'élément métallique est ainsi protégé par une épaisseur de bitume et n'est pas endommagé lors de la mise en place de la couche d'asphalte et/ou d'enrobé. Par ailleurs, grâce à la réalisation d'une profondeur sensiblement constante, la mise en oeuvre d'un procédé de contrôle par radar est possible, afin de connaître la position de cette membrane sous la couche d'asphalte et/ou d'enrobé.

Si cette membrane est de préférence revêtue d'une couche d'asphalte et/ou d'enrobé, cette couche de revêtement peut également être constituée d'un autre matériau, par exemple du béton.

Par géomembrane préfabriquée, il est entendu dans tout ce texte toute membrane en géosynthétique et apparentée telle que définie dans le paragraphe 1.2 de la norme NF EN ISO 10318 de Mai 2006, par exemple un géotextile ou une géogrille pouvant être destinée à être disposée sur des canalisations.

Selon un mode de réalisation préféré, ledit élément métallique est disposé longitudinalement à ladite membrane. Ledit élément métallique est unique.

Ledit élément métallique peut être disposé à environ 20cm d'un bord de la membrane. Ledit élément métallique est constitué d'un galon d'une largeur comprise entre 15 et 20mm.

Grâce à une telle largeur, le revêtement en asphalte et/ou enrobé peut être posé sur la membrane présentant ce galon, sans répercussion sur la tenue de la structure.

Une largeur classique d'une telle membrane ou géomembrane préfabriquée étant de l'ordre d'un mètre, la couche d'étanchéité obtenue comporte alors un tel galon longitudinal tous les mètres. Hors ce galon, un contrôle par radar peut être effectué pour vérifier la structure porteuse, en particulier ses armatures dans le cas d'une structure porteuse en béton armé.

Ledit galon est de préférence un film de polyester métallisé sur ses deux faces.

Ledit métal est avantageusement de l'aluminium.

De préférence, ledit élément métallique est noyé à une profondeur d'environ 1,5mm sous la face supérieure de la membrane.

La membrane ou géomembrane comportant une armature en non-tissé imprégnée à coeur de bitume, une couche supérieure de bitume d'enduction de surface et une couche inférieure de bitume d'enduction de sous-face, ledit élément métallique est avantageusement disposé sur ladite armature.

Il est ainsi obtenu un procédé de fabrication de la membrane ou géomembrane préfabriquée particulièrement simple.

L'invention concerne également une couche d'étanchéité d'un ouvrage de génie civil constituée d'une pluralité de telles membranes ou géomembranes bitumineuses, disposées de façon adjacente.

L'invention est décrite ci-après plus en détail à l'aide de figures ne représentant qu'un mode de réalisation préféré de l'invention.
La figure 1 est une vue représentant une membrane ou géomembrane préfabriquée conforme à l'invention.
La figure 2 est une vue de détail d'une telle membrane ou géomembrane préfabriquée conforme à l'invention
La figure 3 est une vue en coupe transversale d'un tablier de pont pourvu d'une couche d'étanchéité conforme à l'invention.

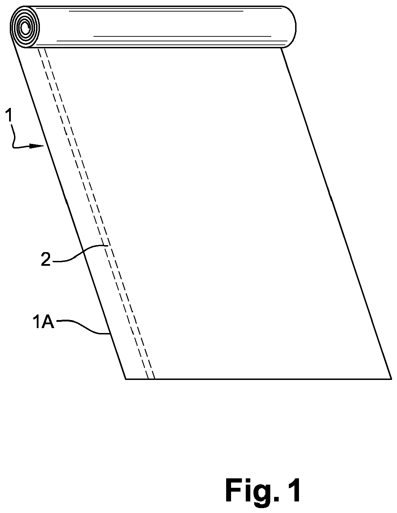

Comme illustré sur la figure 1, une membrane ou géomembrane préfabriquée 1 pour la mise en oeuvre d'un procédé de contrôle par radar conforme à l'invention comporte au moins un élément métallique 2, noyé dans la membrane à une profondeur sensiblement constante, avantageusement à une profondeur d'environ 1,5mm sous la face supérieure de la membrane, et disposé longitudinalement à la membrane.

Très avantageusement, cet élément métallique 2 est unique et peut être disposé à environ 20cm d'un bord 1A de la membrane, qui est le bord de recouvrement de la membrane lors de sa mise en place à côté d'une autre membrane analogue.

De préférence, l'élément métallique est constitué d'un galon d'une largeur comprise entre 1 et 50mm, et avantageusement comprise entre 15 et 20mm.

Ainsi, hors ce galon, un contrôle par radar peut aisément être effectué pour vérifier la structure porteuse, en particulier ses armatures dans le cas d'une structure porteuse en béton, une largeur classique de telle membrane préfabriquée étant de l'ordre d'un mètre.

Le galon 2 est de préférence un film de polyester métallisé par de l'aluminium sur ses deux faces et peut avoir une épaisseur de l'ordre de 12 microns. Selon un exemple de réalisation préféré, le galon 2 présente une largeur de sensiblement 10mm.

A titre d'exemple, un tel film est commercialisé par la société REXOR et est décrit dans la « fiche technique - isolation - N°T4-FT-048 ».

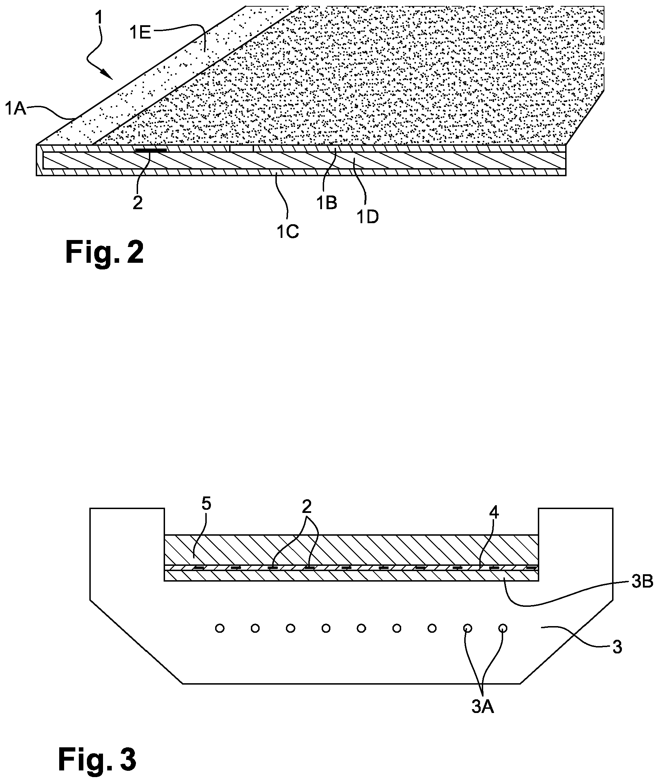

La figure 2 représente plus en détail une telle membrane bitumineuse préfabriquée 1.

La membrane préfabriquée 1 comporte une armature en non-tissé de polyester 1D imprégnée à coeur de bitume, une couche supérieure de bitume d'enduction de surface 1B et une couche inférieure de bitume d'enduction de sous-face 1C.

A titre d'exemple, la membrane a une épaisseur totale de l'ordre de 4mm.

La couche supérieure de bitume d'enduction de surface 1B est revêtu d'une protection minérale, excepté sur une bande 1E latérale le long du bord 1A qui est destiné au recouvrement par une autre membrane analogue mis en place à côté de cette membrane in situ.

A titre d'exemple, cette bande de recouvrement 1E a une largeur d'environ 10cm.

Le galon métallique 2 précédemment décrit est disposé sur l'armature 1D. Il est donc recouvert de la couche supérieure de bitume d'enduction de surface 1B d'une épaisseur de l'ordre de 1,5mm.

Un exemple d'application préférée d'une telle membrane d'étanchéité est illustré sur la figure 3.

Sur un tablier 3 de pont, en béton armé pourvu des armatures référencées 3A, est tout d'abord mis en place une couche de micro-béton 3B de reprofilage destinée à aplanir la surface support de la couche d'étanchéité.

Une couche d'étanchéité 4 est constituée d'une pluralité de N membranes ou géomembranes 1 bitumineuses, disposées de façon adjacentes longitudinalement et avec recouvrement comme il est connu de la technique. Cette couche d'étanchéité 4 présente donc N galons métalliques 2 répartis longitudinalement.

Sur cette couche d'étanchéité 4 est disposée la couche d'asphalte et/ou d'enrobé 5 qui peut être à remettre en état partiellement ou totalement.

Par un contrôle par radar, il peut donc être défini un profil complet, longitudinal et transversal, de la surface supérieure de la couche d'étanchéité 4.

Ainsi, lors du rabotage partiel ou total de la couche d'asphalte et/ou d'enrobé 5, il est possible de définir la profondeur de rabotage, afin d'être assuré de ne pas endommager la couche d'étanchéité 4.

L'invention n'est pas limitée à l'exemple décrit ci-dessus.

Bien que de préférence disposé longitudinalement à la membrane 1, ce galon 2 peut également être disposé transversalement à la membrane 1 ou même de façon inclinée par rapport à son axe longitudinal choisie selon le mode de production de cette membrane 1.

Si l'application décrite est relative à une couche d'étanchéité 4 bitumineuse de tablier de pont, l'invention s'applique également à toute membrane en géosynthétique et apparentée tel que définie dans le paragraphe 1.2 de la norme NF EN ISO 10318 de Mai 2006, par exemple un géotextile ou une géogrille pouvant être destinée à être disposée sur des canalisations.

## Revendications

1. Membrane ou géomembrane bitumineuses (1) préfabriquée pour la mise en oeuvre d'un procédé de contrôle par radar, comportant au moins un élément métallique (2), **caractérisée en ce que** ledit élément métallique (2) est constitué d'un galon (2) unique d'une largeur comprise entre 15 et 20mm noyé dans la membrane.

2. Membrane ou géomembrane selon la revendication précédente, **caractérisée en ce que** ledit élément métallique (2) est disposé longitudinalement à ladite membrane (1).

3. Membrane selon la revendication précédente, **caractérisée en ce que** ledit élément métallique (2) est disposé à environ 20cm d'un bord (1A) de la membrane.

4. Membrane ou géomembrane selon l'une des revendication précédentes, **caractérisée en ce que** ledit galon (2) est un film de polyester métallisé sur ses deux faces.

5. Membrane ou géomembrane selon la revendication précédente, **caractérisée en ce que** ledit métal est de l'aluminium.

6. Membrane ou géomembrane selon l'une des revendications précédentes, **caractérisée en ce que** ledit élément métallique (2) est noyé à une profondeur d'environ 1,5mm sous la face supérieure de la membrane.

7. Membrane ou géomembrane selon l'une des revendications précédentes, comportant une armature en non-tissé (1D) imprégnée à coeur de bitume, une couche supérieure de bitume d'enduction de surface (1B) et une couche inférieure de bitume d'enduction de sous-face (1C), **caractérisée en ce que** ledit élément métallique (2) est disposé sur ladite armature (1D).

8. Couche d'étanchéité (4) d'un ouvrage de génie civil constituée d'une pluralité de membranes ou géomembranes (1) bitumineuses selon l'une des revendications précédentes, disposées de façon adjacente.

## Patentansprüche

1. Bituminöse vorgefertigte Membran oder Geomembran (1) für die Umsetzung eines Radar-Kontrollverfahrens, aufweisend mindestens ein Metallelement (2), **dadurch gekennzeichnet, dass** das Metallelement (2) von einem einzigen Band (2) einer Breite zwischen 15 und 20 mm, eingelassen in die Membran, gebildet ist.

2. Membran oder Geomembran nach vorangehendem Anspruch, **dadurch gekennzeichnet, dass** das Metallelement (2) längs zur Membran (1) angeordnet ist.

3. Membran nach vorangehendem Anspruch, **dadurch gekennzeichnet, dass** das Metallelement (2) zirka 20 cm von einem Rand (1A) der Membran angeordnet ist.

4. Membran oder Geomembran nach einem der vorangehenden Ansprüche, **dadurch gekennzeichnet, dass** das Band (2) eine auf ihren beiden Seiten metallisierte Polyesterfolie ist.

5. Membran oder Geomembran nach vorangehendem Anspruch, **dadurch gekennzeichnet, dass** das Metall Aluminium ist.

6. Membran oder Geomembran nach einem der vorangehenden Ansprüche, **dadurch gekennzeichnet, dass** das Metallelement (2) in einer Tiefe von zirka 1,5 mm unter der oberen Fläche der Membran eingelassen ist.

7. Membran oder Geomembran nach einem der vorangehenden Ansprüche, aufweisend eine Armierung aus Vlies (1 D) mit zentraler Bitumenimprägnierung, eine obere Bitumenbeschichtungsschicht auf der Oberfläche (1 B) und eine untere Bitumenbeschichtungsschicht auf der Unterfläche (1C), **dadurch gekennzeichnet, dass** das Metallelement (2) auf der Armierung (1 D) angeordnet ist.

8. Dichtungsschicht (4) eines Bauwerks, gebildet aus einer Vielzahl von bituminösen Membranen oder Geomembranen (1) nach einem der vorangehenden Ansprüche, die nebeneinander angeordnet sind.

## Claims

1. Bituminous membrane or geomembrane (1) prefabricated for applying a radar control method, including at least one metal element (2), **characterized in that** said metal element (2) consists of a single stripe (2) with a width comprised between 15 and 20 mm, embedded in the membrane.

2. The membrane or geomembrane according to the preceding claim, **characterized in that** said metal element (2) is longitudinally positioned relatively to said membrane (1).

3. The membrane according to the preceding claim, **characterized in that** said metal element (2) is positioned at about 20 cm from one edge (1 A) of the membrane.

4. The membrane or geomembrane according to one of the preceding claims, **characterized in that** said stripe (2) is a polyester film metallized on both of its sides.

5. The membrane or geomembrane according to the preceding claim, **characterized in that** said metal is aluminium.

6. The membrane or geomembrane according to one of the preceding claims, **characterized in that** said metal element (2) is embedded at a depth of about 1.5 mm under the upper face of the membrane.

7. The membrane or geomembrane according to one of the preceding claims, including a non-woven frame (1 D) impregnated to the core with bitumen, an upper surface bitumen coating layer (1 B) and a lower sub-face bitumen coating layer (1 C), **characterized in that** said metal element (2) is positioned on said frame (1 D).

8. A leakproof layer (4) of a civil engineering piece of work consisting of a plurality of bituminous membranes or geomembranes (1) according to one of the preceding claims, positioned in an adjacent manner.
